# EUROPEAN PATENT SPECIFICATION

(11) **EP 1 684 700 B1**
(45) Date of publication and mention of the grant of the patent: **28.03.2007**
(21) Application number: 04764633.6
(22) Date of filing: 30.08.2004
(51) Int. Cl.: A61K 8/18, A61K 47/24

(54) **USE OF PERFLUOROPOLYETHER PHOSPHATES AS STABILIZING AGENTS FOR POLYPHENOLS IN COSMETIC AND/OR DERMATOLOGICAL COMPOSITIONS**
VERWENDUNG VON PERFLUOROPOLYETHER-PHOSPHATEN ALS STABILISIERUNGSMITTEL FÜR POLYPHENOLE IN KOSMETISCHEN UND/ODER DERMATOLOGISCHEN ZUBEREITUNGEN
UTILISATION DES PERFLUOROPOLYETHERS DE PHOSPHATES COMME STABILISANTS DES POLYPHENOLS DANS DES COMPOSITIONS COSMETIQUES ET/OU DERMATOLOGIQUES

(30) Priority: 19.11.2003 EP 03425742
(43) Date of publication of application: 02.08.2006
(73) Proprietor: BIO.LO.GA. S.r.l., 31015 Conegliano (IT)
(72) Inventor: PANIN, Giorgio, I-45100 Rovigo (IT)
(74) Representative: Ferreccio, Rinaldo
(86) International application number: PCT/EP2004/009667
(87) International publication number: WO 2005/049089

(56) References cited:
- EP-A- 0 390 206
- EP-A- 1 074 243
- DE-A- 19 845 266
- DE-A- 19 845 271
- DE-A- 19 845 305
- FR-A- 2 773 811

## Description

### Field of application

The present invention refers, in general, to the cosmetic sector of industry.

The invention concerns, in particular cosmetic or dermatological compositions containing polyphenols stabilized by PFPE phosphates and the use of perfluoropolyether (PFPE) phosphates as stabilizing agents for polyphenols.

### State of the Known Art

The polyphenols constitute a very vast family of natural substances of vegetal origin, comprising various sub-families like the flavones, flavanones, flavonoids and isoflavones. The first indication concerning the possible biological nutritional and pharmacological function of polyphenols came from Szednt-Gyorgy, the discoverer of Vitamin C, who observed that polyphenols protect Vitamin C from oxidation.

Therefore it has been the antioxidant function of polyphenols that has been the thread leading to very active research over the last fifty years. Even though there has never been found any proof of a vitamin nature Tor the polyphenols, and hence no characteristic of essentiality in the prevention of a specific syndrome connected with vitamin insufficiency, the marked antioxidant activity shown by many polyphenols has stimulated studies that have resulted in the expansion of their practical application in fields ranging from that of alimentation to those of nutraceutics and cosmetics.

In fact it is generally accepted today that polyphenols reduce the risk of chronic-degenerative diseases (including cancer) through a series of molecular mechanisms directly or indirectly connected with antioxidant activity.

In this context one can also include tocopherol (Vitamin E) that, despite its specific vitamin activity on the model of fetal reabsorption, must be considered as a polyphenol of vegetal origin, its assumption reducing the risk of specific chronic degenerative diseases through a mechanism that is, though not exclusively, the antioxidant type.

In general terms antioxidants act in a physio-pathological condition called "oxidative stress", where the balance between the oxidants and the molecules that oppose oxidation favors the former. Oxidants are, in general, free radicals produced by oxygen metabolism under varying conditions, from irradiation with ionizing or UV radiation, to exposure to toxic agents or polluting conditions and inflammation.

Antioxidants interrupt the chain of harmful events at various levels: intercepting the primary oxidant species and those species propagating reaction chains, and governing cell response to the initial lesion. In fact, biological damage from oxidative stress is mostly manifested as a "reaction to the lesion", with a reprogramming of the genic expression of the interested cells. Modified biological behavior, including inflammation and apoptosis, is part of the phenomenology of the damage that can be demonstrated clinically. Skin is a tissue particularly exposed to oxidative stress. In addition to specific inflammatory phenomena, similar to those in other organs, skin is exposed to environmental stimuli that have, generally speaking, the characteristics of oxidants. In fact, UV radiation and a great many environmental pollutants, particularly if subjected to redox transitions like, for example, metals, produce oxidation through an oxyradical mechanism.

The biological reaction to continuous radiation and/or environmental oxidative damage is the first cause of skin aging where the continuously irritating stimulus produces a cell reaction that leads to, in addition to inflammation, protease activation that, when the mechanisms of continuous damage repair are not functionally optimal, finally causes atrophy of the connective tissue matrix and wrinkle formation.

The biological antioxidant defence mechanisms of the skin comprise enzymatic and intracellular chemical systems to which a particular vitamin E cycle is added. In fact the vitamin is secreted with the sebum, and this secretion is stimulated by irritative oxidant stimuli like, for example, UV radiation. From the superficial sebaceous layer the vitamin E is therefore reabsorbed through the corneous layer to different deeper depths, so it can be deduced that there is a function also at an extracellular level. Experimental evidence therefore suggests that also physiologically there is a mechanism of protection based on "topic" antioxidant use.

The topic use of antioxidants in cosmetology and in slowing down skin aging is supported by the molecular mechanism of the damage and of the antioxidants.

Topically administered antioxidants can be expected to:
1) block the initiating oxidant species;
2) block the progression of the oxidative reaction chain;
3) "control" the inflammatory reaction;
4) modulate the genic reprogramming responsible for possibly damaging response;
5) inhibit the proteases that degrade the connective tissue matrix;
6) promote cell and tissue repair mechanisms including revascularization.

To these chemical or biochemical effects there are associated, for different antioxidants, in particular topical formulations, physical effects like the quenching of electronically excited species, the absorption of UV radiation and the prevention of transdermal water loss.

Polyphenols are able to exert the beneficial effects illustrated above but they have the inconvenience of marked instability in the dermatological and/or cosmetic compositions containing them.

In the prior art different methods are proposed to stabilize polyphenols. For example, patent application EP 995 432 suggests the use of surfactants constituted by mono-, oligo- and poly-ethers, esters and ether-esters with alkyl or alkenyl or hydroxyacyl groups with 10-30 carbon atoms to stabilize the flavones, flavanones and/or flavonoids against photochemical and/or oxidative degradation.

For the same aim other documents describe the use of an amphiphilic phyllosilicate (EP 1 200 042), of nitrylotriacetic acid (EP 995 422), of 2-tert-butylhydroquinone (EP 997 133), of saturated fatty acid mono- and/or diglyceride esters partly neutralized with citric acid (EP 1 000 603), of an alkylglucoside tensioactive (EP 998 898) and of butylhydroxytoluol (EP 998 899).

### Summary of the invention

The problem at the base of the present invention is to provide a new stabilizing agent for the polyphenols, allowing the achievement of cosmetic or dermatological formulations for topical use, in which the polyphenols are stable and active throughout the whole conservation period normally required for such products that is at least 36 months.

Such a problem has been solved using as the said polyphenol stabilizing agent at least one perfluoropolyether phosphate (PFPE phosphate), in particular a perfluoropolyether phosphate of formula (I):

R_{f}-[CF₂CH₂-O-(CHR₁-CHR₂O)ₙ-P(O)(OH)₂]ₓ (I)

wherein
x = 1 or 2;
R₁ and R₂ are independently selected from H and CH₃;
n is an integer between 1 and 50, preferably 1-6;
R_{f} is a perfluoropolyether chain with a number average molecular weight between 400 and 1800, preferably 500-1300, comprising repeating units chosen from among the following:
a) -(C₃F₆O)-
b) -(CF₂CF₂O)-
c) -(CFL₀O)-, wherein L₀ = -F, -CF₃;
d) -CF₂(CF₂)_{y}CF₂O-, wherein y = 1 or 2;
e) -CH₂CF₂CF₂O-,
and wherein, when x = 1, an end group is a perfluoroalkyl selected from CF₃O, C₂F₅O, C₃F₇O.

Particularly preferred are the perfluoropolyether phosphates of formula (I) in which R_{f} has one of the following structures:

1) -(CF₂O)ₐ-(CF₂CF₂O)_{b}-

wherein b/a is between 0.3 and 10 and a is an integer different from 0;

2) -(CF₂-(CF₂)_{y}-CF₂O)_{b'}-

wherein y = 1 or 2;

3) -(C₃F₆O)ᵣ-(C₂F₄O)_{b}-(CFL₀O)ₜ-,

wherein r/ b = 0.5-2.0, (r+b)/t = 10-30, b and t are integers different from 0;

4) -(OC₃F₆)ᵣ-(CFL₀O)ₜ-OCF₂-R'_{f}-CF₂O-(C₃F₆O)ᵣ-(CFL₀O)ₜ-

5) -(CF₂CF₂CH₂O)_{q'}-R'_{f}-O-(CH₂CF₂CF₂O)_{q}'-

where R'_{f} is a fluoroalkylene group with 1-4 carbon atoms;
Lo is selected between F and CF₃;

6) -(C₃F₆O)ᵣ-OCF₂-R'_{f}-CF₂O-(C₃F₆O)ᵣ-

wherein in the above formulas:
-(C₃F₆O)- represents units of formula:
-(CF(CF₃)CF₂O)- and/or -(CF₂-CF(CF₃)O)-
a, b, b', q', r, t are integers whose sum is such that R_{f} shows values of number average molecular weight Mₙ that fall between about 400 and 1800, preferably between 500 and 1300.

Particularly advantageous to the goals of the present invention is the use of perfluoropolyether diphosphates of the general formula (II):

-CF₂-O(CF₂CF₂O)_{b}(CF₂O)ₐ-CF₂-[CH₂-(OCH₂CH₂)ₙO-PO(OH)₂]₂ (II)

wherein n = 1 or 2, b/ a = 0.5-3.0 and a, b and r have the above reported meanings.

The above perfluoropolyether phosphates and diphosphates are known from patent applications EP 1 074 243 and EP 1 145 722, that respectively describe their use as cosmetic composition ingredients of high water- and oil-repellency and as preservatives in formulas for topical use, without mentioning any stabilizing effect with regard to polyphenols, to vitamin E or to other compounds with antioxidant activity.

Using the said perfluoropolyether diphosphates as stabilizing agents, there have been provided, according to the present invention, cosmetic and/or dermatological compositions for topical use comprising as the active substance polyphenols associated with a suitable carrier, characterized by containing, as the stabilizing agent, an effective amount of a perfluoropolyether phosphate, in particular a perfluoropolyether diphosphate according to the formula (II).

Preferably, such cosmetic and/or dermatological compositions contain a perfluoropolyether diphosphate amount of between 0.1 and 5.0% in weight of total composition weight and conveniently from 0.2 to 1.0%.

The polyphenol content is preferably between 0.1% and 5% by weight of the total composition weight and conveniently from 0.2 to 2%.

The Applicant has furthermore ascertained that perfluoropolyether diphosphates stabilize, very efficiently, another natural antioxidant, vitamin E, also in its non-esterifted form.

Therefore the present invention concerns, furthermore, cosmetic and/or dermatological compositions for topical use containing, as active substances, one or more polyphenols and vitamin E, together with a suitable carrier, characterized by containing as stabilizing agent an effective amount of a perfluoropolyether diphosphate according to the formula (II).

Vitamin E can be used as d-α-tocopherol or as a mixture of the two enantiomers d and 1 of the α-tocopherol or as a mixture of other tocopherols (β, γ, ε, ζ, η) of vegetal origin or as tocotrienol. The different forms of vitamin E can be natural or synthetic in origin.

The amount of perfluoropolyether diphosphate contained in such compositions is that indicated above.

Different vegetal polyphenols and Vitamin E can sustain, with different efficiency, protection mechanisms against free radicals and oxidative stress, so for this reason the association of one or more polyphenols with vitamin E is particularly effective.

On the basis of the specific reactivity of the different polyphenols it is foreseen that particularly efficient skin protection should result from a mixture of a) Vitamin E in the free form; b) one or more polyphenols, like, for example, a standard extract of grape seed rich in polymeric procyanidins and an extract of green tea rich in epigallocatechin gallate.

A topical preparation using these components is very difficult to obtain for two reasons: the different lipophilicity/hydrophilicity and solubility of the components and the susceptibility to oxidation, this last an obvious and inevitable consequence of the high antioxidant activity.

The redox chemistry of phenolic antioxidants foresees that autoxidation begins with the extraction of an atom of phenolic hydrogen or, far more easily, with a one electron transition permitted by the acid dissociation of the phenol. This reaction is furthermore favored by electron acceptors like transition metals and the presence of water at the reaction site.

Therefore the stabilization of the polyphenols and vitamin E for a cosmetological preparation requires an environment in which the phenol groups are not exposed to water, and an acidity that prevents their dissociation.

The PFPE diphosphate is an acid ester as it is obtained by monosubstitution of orthophosphoric acid. The acidity of the most active hydrogen acid is even higher than that of the hydrogen with the greatest protonic characteristics of phosphoric acid, as is demonstrated by the comparison between the corresponding dissociation constant values (PFPE phosphate PKₐ = 1.84 ; orthophosphoric acid pKₐ= 2.15).

PFPE diphosphate is very soluble in alcohols and glycols, also in a ratio of 1:1. Unexpectedly the addition of water to a concentrated solution of PFPE phosphate and alcohol or glycol leads to the formation of limpid solutions with an acidic pH; in concentrations between 0.5 and 5% they have a pH of 2.0-3.5. As demonstrated by numerous dermatological patch tests, these solutions or the emulsions that contain them have, despite the acidity, been shown to be non-irritating at the cutaneous level and even capable of reducing the cutaneous irritation of other substances or other acids like the alpha-hydroxyacids.

Therefore acidifying the emulsion with PFPE phosphates results in a preparation, not irritating at the cutaneous level, in which the polyphenol substances present for example in the extract of green tea or grape seeds are, like tocopherol, stabilized.

The optimum stability demonstrated, also under heating, by the emulsion in question leads to the supposition of a stabilizing mechanism that is not simply due only to the acid environment, but also to an active role of the fluorinated substance, which is able to protect the vegetal extracts and tocopherol, due to the perfluoropolyether chain having a screening effect with regard to the phenolic molecules.

Furthermore the compositions according to the present invention can optionally contain other compounds endowed with antioxidant or else vitaminic activity and particularly susceptible to oxidation, like for example vitamin A, carotenes, carotenoids, lutein, lycopene and xanthophylls.

It is to be noted that even free ascorbic acid is surprisingly stabilized by the perfluoropolyether phosphates, even though it is notoriously unstable in the usual cosmetic and dermatological formulations.

The stabilization of ascorbic acid is achieved with the use of perfluoropolyether phosphates according to the invention, in particular with those according to formula (II), in the percentage amounts reported above in relation to polyphenol stabilization.

### Detailed description of a preferred embodiment

The present invention will be further described making reference to several examples of formulations according to the present invention, supplied here as illustrative and not limiting, in which the different ingredients are indicated with the respective name INCI and in percentages of weight on total weight.

### EXAMPLE 1

| | |
|---|---|
| Steareth-2 | 4 |
| Steareth-21 1 | 4 |
| Cetearyl alcohol | 4 |
| Glyceryl stearate | 3 |
| Octyldodecanol | 3 |
| Dimethicone | 0.5 |
| Tocopherol | 5 |
| Glycerin | 8 |
| Pentylene Glycol | 7 |
| Disodium EDTA | 0.05 |
| Polyperfluoethoxymethoxy-difluoroethyl PEG phosphate¹ | 0.5 |
| Camelia sinensis² | 0.5 |
| Vitis vinifera³ | 0.5 |
| Aqua | q.b. a 100 |

1) Use was made of the commercial product Fomblin HC/P2-1000^{®} of the company Solvay Solexis.
2) Use was made of the Indena company product Greenselect^{®}, a stabilized extract of green tea.
3) Use was made of the Indena company product Leucoselect^{®}, a stabilized extract of grape seeds.

A cream was prepared by putting into an emulsifier first the water and the water soluble ingredients, except for Greenselect^{®} and Leucoselect^{®} and then all the oily and liposoluble ingredients, except tocopherol (free vitamin E) and heat was applied until the fats had melted completely.

Successively vitamin E was introduced and the vacuum pump activated to obtain a pressure of 40 cmHg. At this point the turbine and the mixer of the emulsifier were put into action at maximum speed for 10-15 minutes, after which cooling to room temperature took place; with the turbine turned off the polyphenols (Greenselect^{®} and Leucoselect^{®}) were added, and mixed in for several minutes.

The thus obtained O/W cream was beige-orange in color, with pH 3.7, a viscosity (10 rpm) of 32,000 mPs s and stable at a centrifugation of 6000 rpm for 30 minutes.

The cream was packed in plastics containers of 50 ml and subjected to a test to confirm the stability. A series of samples of the cream contained in the said small plastic jars was kept in an oven at 45°C for twelve weeks, after which the viscosity of the cream was checked and found unaltered with respect to the starting value.

In addition there was the verification of the amount of polyphenols and vitamin E present in the cream at the end of the twelve weeks at 45°C, by means of an HPLC method, using a Macherey-Nagel column, Nucleosil 100-5 C18, packed with 5 µm particles (150 x 4.6 mm internal diameter), and a pre-column system Nucleosil 100-5 C18, CC 8/4.

The determination of free tocopherol, carried out using methanol as the eluent in isocratic conditions, with a flow rate of 1 ml/min and λ 290 nm, gave a value of 5.2%.

The determination of the polyphenol concentration in the cream was carried out using a mobile phase in binary gradient as eluent, composed of: phase A = 0.3% formic acid in water and phase B = methanol; flow rate 1 ml/min; λ = 278 nm. In such conditions the Greenselect^{®} has a retention time Rₜ = 22.84 min and the Leucoselect^{®} Rt = 6.53 min.

The above determination supplied a value of 0.50% for Greenselect^{®} and 0.49% for Leucoselect^{®}.

As can be easily ascertained from the above reported data, the concentration of the three active ingredients (vitamin E and the two polyphenols) remained substantially unaltered after a twelve week period at 45°C. This confirms that the presence of the perfluoropolyether diphosphate in the cream stabilized both the vitamin E and the polyphenols with regard to oxidative degradation.

### EXAMPLE 2

| | |
|---|---|
| Steareth-2 | 4 |
| Steareth-21 | 2 |
| Cetearyl alcohol | 5 |
| Glyceryl stearate | 6 |
| Octyldodecanol | 6 |
| Dimethicone | 0.5 |
| Glycerin | 6 |
| Pentylene Glycol | 7 |
| Disodium EDTA | 0.06 |
| Polyperfluoethoxymethoxy-difluoroethyl PEG phosphate¹ | 0.8 |
| Camelia sinensis² | 1.0 |
| Vitis vinifera³ | 1.0 |
| Aqua | q.b. a 100 |

For the references 1, 2 and 3 see what is reported in example 1.

An O/W cream was prepared starting with the ingredients listed above and proceeding in a way analogous to example 1.

The thus obtained O/W cream was beige-orange in color, had a pH of 3.8, a viscosity (10 rpm) of 31,000 mPs s and was stable to centrifugation at 6000 rev/min for 30 minutes.

The cream, subjected to the same stability verification test described in example 1, was shown to be perfectly stable and to conserve, substantially unaltered, the polyphenol content.

### EXAMPLE 3

| | |
|---|---|
| Steareth-2 | 3 |
| Steareth-21 | 4 |
| Cetearyl alcohol | 4 |
| Glyceryl stearate | 3 |
| Octyldodecanol | 3 |
| Dimethicone | 0.5 |
| Tocopherol | 4 |
| Glycerin | 8 |
| Pentylene Glycol | 7 |
| Ascorbic acid | 2 |
| Disodium EDTA | 0.08 |
| Polyperfluoethoxymethoxy-difluoroethyl PEG phosphate¹ | 1.0 |
| Camelia sinensis² | 0.8 |
| Vitis vinifera³ | 0.8 |
| Aqua | q.b. a 100 |

For the references 1, 2 and 3 see what is reported in example 1.

An O/W cream was prepared starting with the ingredients listed above and proceeding in a way analogous to example 1.

The thus obtained O/W cream was beige-orange in color, had a pH of 3.6, a viscosity (10 rpm) of 32,000 mPs s and was stable to centrifugation at 6000 rev/min for 30 minutes.

The cream, subjected to the same stability verification test described in example 1, was shown to be perfectly stable and to conserve, substantially unaltered, the content of the polyphenols and vitamin E.

The stability of the ascorbic acid contained in the cream was determined as follows.

A series of samples of the cream contained in the said small plastic jars was kept for a period of 4 months at room temperature and then in an oven at 45°C for one month, after which the ascorbic acid content of the cream was checked.

In order to determine the ascorbic acid content, 100 mg of cream were diluted in 100 ml of 0.1 M phosphate buffer at pH 6, and the thus obtained suspension was magnetically stirred in the dark for 20 minutes. Thereafter, 30 ml of the suspension were transferred to a 50 ml centrifuge tube and 15 ml of chloroform were added. The tube was shaken for 10 minutes and then centrifuged at 6000 rpm for 5 minutes. The upper aqueous phase was directly analyzed with a UV Vis Jasco V-350 spectrophotometer, using phosphate buffer as a blank and the ascorbic acid peak at 266.5 nm was measured.

The ascorbic acid concentration was determined by means of a calibration curve made from standard solutions containing 1.0, 1.5 and 2.0 mg ascorbic acid/ 100 ml phosphate buffer.

The absorbance value obtained for a solution of 2 mg of ascorbic acid in 100 ml phosphate buffer was 1.619 and the value obtained for the sample prepared as described above was 1.492. Based on the calibration curve, the latter value corresponded to an ascorbic acid concentration of 1.844 mg/ 100 ml.

This means that only 7.8% of ascorbic acid was lost by the end of the storage period as specified above.

## Claims

1. Use of perfluoropolyether phosphates as stabilizing agents for polyphenols in cosmetic and/or dermatological compositions for topical application.

2. Use according to claim 1, wherein said perfluoropolyether phosphates have the general formula (I):
R_{f}-[CF₂CH₂-O-(CHR₁-CHR₂O)ₙ-P(O)(OH)₂]ₓ (I)
wherein
x = 1 or 2;
R₁ and R₂ are independently selected between H and CH₃;
n is an integer between 1 and 50, preferably 1-6;
R_{f} is a perfluoropolyether chain with a number average molecular weight between 400 and 1800, preferably 500-1300, comprising repeating units selected from the following:
a) -(C₃F₆O)-
b) -(CF₂CF₂O)-
c) -(CFL₀O)-, wherein L₀ = -F, -CF₃;
d) -CF₂(CF₂)_{y}CF₂O-, wherein y = 1 or 2;
e) -CH₂CF₂CF₂O-,
and wherein, when x = 1, an end group is a perfluoroalkyl selected from CF₃O, C₂F₅O, C₃F₇O.

3. Use according to claim 2, wherein R_{f} has one of the following structures:
1) -(CF₂O)ₐ-(CF₂CF₂O)_{b}-
wherein b/a lies between 0.3 and 10 and a is an integer different from 0;
2) -(CF₂-(CF₂)_{y}-CF₂O)_{b}-
wherein y = 1 or 2;
3) -(C₃F₆O)ᵣ-(C₂F₄O)_{b}-(CFL₀O)ₜ-,
wherein r/ b = 0.5-2.0, (r+b)/t = 10-30, b and t are integers different from 0;
4) -(OC₃F₆)ᵣ-(CFL₀O)ₜ-OCF₂-R'_{f}-CF₂O-(C₃F₆O)ᵣ-(CFL₀O)ₜ-
5) -(CF₂CF₂CH₂O)_{q'}-R'_{f}-O-(CH₂CF₂CF₂O)_{q'}-
wherein R'_{f} is a fluoroalkylene group with 1-4 carbon atoms;
Lo is chosen between F and CF₃;
6) -(C₃F₆O)ᵣ-OCF₂-R'_{f}-CF₂O-(C₃F₆O)ᵣ-
wherein in the above formulas:
-(C₃F₆O)- represents units of formula:
-(CF(CF₃)CF₂O)- and/or -(CF₂-CF(CF₃)O)-
a, b, b', q', r, t are integers, whose sum is such that R_{f} has values of number average molecular weight Mₙ lying between about 400 and about 1800, preferably between 500 and 1300.

4. Use according to claim 3, wherein the perfluoropolyether phosphates are perfluoropolyether diphosphates of formula (II):
-CF₂-O(CF₂CF₂O)_{b}(CF₂O)ₐ-CF₂-[CH₂-(OCH₂CH₂)ₙO-PO(OH)₂]₂ (II)
wherein n = 1 or 2, b/a = 0.5-3.0 and a, b and r have the meanings reported in claim 3.

5. A cosmetic and/or dermatological composition for topical use comprising, as active substances, polyphenols in association with a suitable carrier, **characterized by** containing , as stabilizing agent, an effective amount of at least one perfluoropolyether phosphate.

6. A cosmetic and/or dermatological composition according to claim 5, wherein said stabilizing agent is a perfluoropolyether diphosphate according to formula (II) of claim 4.

7. A composition according to claim 6, wherein said at least one perfluoropolyether diphosphate is contained in an amount included between 0.1 and 5.0% by weight of total composition weight.

8. A composition according to claim 7, wherein said at least one perfluoropolyether diphosphate is contained in an amount included between 0.2 and 1.0% by weight of total composition weight.

9. A composition according to any one of claims 5 to 8, wherein the polyphenol content is included between 0.1% and 5% by weight of total composition weight.

10. A composition according to any one of claims 5 to 9, further including vitamin E.

11. A composition according to claim 10, wherein said vitamin E is contained in an amount between 0.5 and 10% by weight of total composition weight.

12. A composition according to any one of claims 5 to 11, further including ascorbic acid.

13. A composition according to claim 12, wherein the ascorbic acid is contained in an amount between 0.1 and 10% by weight of total composition weight.

14. A composition according to any one of claims 5 to 13, further including at least one compound selected from the group consisting of vitamin A, carotenes, carotenoids, lutein, lycopene and xanthophylls.

15. A composition according to any one of claims 5 to 14, **characterized by** being in the form of a cream.

## Patentansprüche

1. Verwendung von Perfluorpolyetherphosphaten als stabilisierende Mittel für Polyphenole in kosmetischen und/oder dermatologischen Zusammensetzungen zur topischen Anwendung.

2. Verwendung gemäß Anspruch 1, wobei die Perfluorpolyetherphosphate die allgemeine Formel (I) aufweisen:
*R_{f} -*[*CF*₂*CH*₂ *-O-* (*CHR*₁*- CHR*₂*O*)*ₙ -P(O)(OH)*₂]*ₓ* (I)
wobei
x = 1 oder 2;
R₁ und R₂ unabhängig ausgewählt sind zwischen H und CH₃;
n eine ganze Zahl zwischen 1 und 50, vorzugsweise 1-6 ist;
R_{f} eine Perfluorpolyether-Kette mit einer durchschnittlichen Anzahl des Molekulargewichts zwischen 400 und 1800, vorzugsweise 500-1300 ist, die wiederholte Einheiten aufweist, die aus den Folgenden ausgewählt sind:
a) *-* (*C*₃*F*₆*O*)*-*
b) *-* (*CF*₂*CF*₂*O*)*-*
c) -(*CFL*₀*O*)-*,* wobei *L*₀ = *-F,-CF*₃*;*
d) *- CF*₂(*CF*₂)_{y} *CF*₂*O* -, **wobei** y = 1 oder 2;
e) *- CH*₂*CF*₂*CF*₂*O-*,
und wobei, wenn x = 1, eine Endgruppe ein Perfluoralkyl ist, das ausgewählt ist aus *CF*₃*0, C*₂*F*₅*O, C*₃*F*₇*O.*

3. Verwendung gemäß Anspruch 2, wobei R_{f} eine der folgenden Strukturen aufweist:
1) *-* (*CF*₂*O*)*ₐ -* (*CF*₂*CF*₂*O*)*_{b} -*
wobei b/a zwischen 0,3 und 10 liegt und a eine ganze Zahl ungleich 0 ist;
2) *-* (*CF*₂ *-* (*CF*₂)*_{y} - CF*₂*O*)*_{b}, -*
wobei y = 1 oder 2;
3) *-* (*C*₃*F*₆*O*)*ᵣ -* (*C*₂*F*₄*O*)*_{b} -*(*CFL*₀*O*)*ₜ-,*
wobei r/b = 0,5-2,0, (r+b)/t = 10-30, b und t ganze Zahlen und ungleich 0 sind;
4) -(*OC*₃*F*₆)*ᵣ -*(*CFL*₀*O*)*ₜ -OCF*₂ *-R_{f} -CF*₂*O-*(*C*₃*F*₆*O)ᵣ -(CFL*₀*O)ₜ-*
5) *-* (*CF*₂ *CF*₂*CH*₂*O)_{q'} - R'_{f} -*O*-*(*CH*₂*CF*₂*CF*₂*O*)*_{q'}-*
wobei *R'_{f}* eine Fluoralkylen-Gruppe mit 1-4 Kohlenstoffatomen ist; L₀ wird zwischen F und CF₃ gewählt;
6) *-* (*C*₃*F*₆*O)ᵣ - OCF*₂*-R'_{f} -CF*₂*O-*(*C*₃*F*₆*O)ᵣ-*
wobei in den oben genannten Formeln:
*-* (*C*₃*F*₆*O*) - Einheiten der Formel:
-(*CF*(*CF*₃)*CF*₂*O*)- und/oder *-*(*CF*₂*CF*(*CF*₃)*O-* darstellt;
a, b, b', q', r, t ganze Zahlen sind, deren Summe so gewählt wird, dass R_{f} Zahlenwerte des durchschnittlichen Molekulargewichts Mₙ aufweist, die zwischen ca. 400 und ca. 1800, vorzugsweise zwischen 500 und 1300 liegen.

4. Verwendung gemäß Anspruch 3, wobei die Perfluorpolyetherphosphate Perfluorpolyetherdiphosphate der Formel (II) sind:
*-CF*₂*O*(*CF*₂*CF*₂*O*)*_{b}*(*CF*₂*O*)*ₐ-CF*₂*-* [*CH*₂*-*(*OCH*₂*CH*₂)*ₐO-PO*(*OH*)₂]₂ (II)
wobei n = 1 oder 2, b/a = 0,5-3,0 und a, b und r die in Anspruch 3 genannten Bedeutungen aufweisen.

5. Kosmetische und/oder dermatologische Zusammensetzung zur topischen Anwendung umfassend, als aktive Substanzen, Polyphenole in Verbindung mit einem geeigneten Träger, charakterisiert durch das Enthalten einer aktiven Menge von wenigstens einem Perfluorpolyetherphosphat als stabilisierendes Mittel.

6. Kosmetische und/oder dermatologische Zusammensetzung gemäß Anspruch 5, wobei das stabilisierende Mittel ein Perfluorpolyetherdiphosphat gemäß Formel (II) des Anspruchs 4 ist.

7. Zusammensetzung gemäß Anspruch 6, wobei wenigstens eines der Perfluorpolyetherdiphosphate in einer Menge zwischen 0,1 und 5,0 Gewichts-% des Gesamtgewichts der Zusammensetzung enthalten ist.

8. Zusammensetzung gemäß Anspruch 7, wobei wenigstens eines der Perfluorpolyetherdiphosphate in einer Menge zwischen 0,2 und 1,0 Gewichts-% des Gesamtgewichts der Zusammensetzung enthalten ist.

9. Zusammensetzung gemäß einem der Ansprüche 5 bis 8, wobei der Polyphenolgehalt zwischen 0,1 und 5 Gewichts-% des Gesamtgewichts der Zusammensetzung liegt.

10. Zusammensetzung gemäß einem der Ansprüche 5 bis 9, ferner umfassend Vitamin E.

11. Zusammensetzung gemäß Anspruch 10, wobei Vitamin E in einer Menge zwischen 0,5 und 10 Gewichts-% des Gesamtgewichts der Zusammensetzung enthalten ist.

12. Zusammensetzung gemäß einem der Ansprüche 5 bis 11, ferner umfassend Ascorbinsäure.

13. Zusammensetzung gemäß Anspruch 12, wobei Ascorbinsäure in einer Menge zwischen 0,1 und 10 Gewichts-% des Gesamtsgewichts der Zusammensetzung enthalten ist.

14. Zusammensetzung gemäß einem der Ansprüche 5 bis 13, ferner umfassend wenigstens einem der Inhaltsstoffe, ausgewählt aus der Gruppe bestehend aus Vitamin A, Carotinen, Carotinoiden, Lutein, Lycopin und Xanthophyllen.

15. Zusammensetzung gemäß einem der Ansprüche 5 bis 14, charakterisiert durch die Darreichungsform einer Creme.

## Revendications

1. Utilisation des phosphates de perfluoropolyéther comme agents stabilisants des polyphénols dans les compositions cosmétiques et/ou dermatologiques pour application locale.

2. Utilisation selon la revendication 1, dans laquelle lesdits phosphates de perfluoropolyéther sont de formule générale (I) :
R_{f}-[CF₂CH₂-O-(CHR₁-CHR₂O)ₙ-P(O)(OH)₂]ₓ (I)
où
x = 1 ou 2;
R₁ et R₂ sont choisis indépendamment entre H et CH₃ ;
n est un entier entre 1 et 50, de préférence 1-6 ;
R_{f} est une chaîne perfluoropolyéther ayant un poids moléculaire moyen compris entre 400 et 1 800, de préférence 500-1 300, comprenant des unités répétitives choisies parmi les éléments suivants :
a)-(C₃F₆O)-
b) -(CF₂CF₂O)-
c) -(CFL₀O)-, où L₀ = -F, -CF₃ ;
d) -CF₂(CF₂)_{y}CF₂O-, où y = 1 ou 2 ;
e) -CH₂CF₂CF₂O-,
et où, lorsque x = 1, un groupe à l'extrémité ou terminal est un perfluoroalkyle choisi parmi CF₃O, C₂F₅O, C₃F₇O.

3. Utilisation selon la revendication 2, où R_{f} a l'une des structures suivantes :
1) -(CF₂O)ₐ-(CF₂CF₂O)_{b}-
où b/a est compris entre 0,3 et 10 et est un entier différent de 0 ;
2) -(CF₂-(CF₂)_{y}-CF₂O)_{b}-
où y=1 ou 2;
3) -(C₃F₆O)ᵣ-(C₂F₄O)_{b}-(CFL₀O)ₜ-
où r/b = 0,5-2,0, (r+b)/t= 10-30, b et t sont des entiers différents de 0 ;
4) -(OC₃F₆)ᵣ-(CFL₀O)ₜ-OCF₂-R'_{f}-CF₂O-(C₃F₆O)ᵣ-(CFL₀O)ₜ-
5) (CF₂CF₂CH₂O)_{q},-R'_{f}-O-(CH₂CF₂CF₂O)_{q'}-
où R'_{f} est un groupe fluoroalkylène ayant 1-4 atomes de carbone ;
L₀ est choisi entre F et CF3 ;
6) -(C₃F₆O)ᵣ-OCF₂-R'_{f}-CF₂O-(C₃F₆O)ᵣ-
où, dans les formules ci-dessus :
-(C₃F₆0)- représente des unités de formule :
-(CF(CF₃)CF₂O)- et/ou -(CF₂-CF(CF₃)O)-
a, b, b', q', r, t sont des entiers dont la somme est telle que R_{f} a des valeurs de poids moléculaire moyen Mₙ compris entre environ 400 et environ 1 800, de préférence entre 500 et 1 300.

4. Utilisation selon la revendication 3, dans laquelle les phosphates de perfluoropolyéther sont des diphosphates de perfluoropolyéther de formule (II) :
-CF₂-O(CF₂CF₂O)_{b}(CF₂O)ₐ-CF₂-[(CH₂-(OCH₂CH₂)ₙO-PO(OH)₂)ₕ (II)
où n = 1 ou 2, b/a = 0,5-3,0 et a, b, et r ont les significations indiquées à la revendication 3.

5. Composition cosmétique et/ou dermatologique pour utilisation locale comprenant, comme substances actives, des polyphénols en association avec un vecteur approprié, **caractérisée en ce qu'**elle contient, comme agent stabilisant, une quantité efficace d'au moins un phosphate de perfluoropolyéther.

6. Composition cosmétique et/ou dermatologique selon la revendication 5, dans laquelle ledit agent stabilisant est un diphosphate de perfluoropolyéther de formule (II) selon la revendication 4.

7. Composition selon la revendication 6, dans laquelle ledit au moins diphosphate de perfluoropolyéther est contenu en un volume compris entre 0,1 et 5,0 % en poids du poids total de la composition.

8. Composition selon la revendication 7, dans laquelle ledit au moins diphosphate de perfluoropolyéther est contenu en un volume compris entre 0,2 et 1,0 % en poids du poids total de la composition.

9. Composition selon l'une quelconque des revendications 5 à 8, dans laquelle le volume de polyphénol est contenu en un volume compris entre 0,1 et 5 % en poids du poids total de la composition.

10. Composition selon l'une quelconque des revendications 5 à 9, comprenant en outre de la vitamine E.

11. Composition selon la revendication 10, dans laquelle ladite vitamine E est contenue en un volume compris entre 0,5 et 10 % en poids du poids total de la composition.

12. Composition selon l'une quelconque des revendications 5 à 11, comprenant en outre de l'acide ascorbique.

13. Composition selon la revendication 12, dans laquelle l'acide ascorbique est contenu en un volume compris entre 0,1 et 10 % en poids du poids total de la composition.

14. Composition selon l'une quelconque des revendications 5 à 13, comprenant en outre au moins un composé choisi parmi le groupe constitué par la vitamine A, les carotènes, les caroténoïdes, la lutéine, le lycopène et les xantophylles.

15. Composition selon l'une quelconque des revendications 5 à 14, **caractérisée en ce qu'**elle est sous la forme d'une crème.
